# EUROPÄISCHE PATENTSCHRIFT

(11) **EP 4 401 667 B1**
(45) Veröffentlichungstag und Bekanntmachung des Hinweises auf die Patenterteilung: **19.11.2025**
(21) Anmeldenummer: 23716616.0
(22) Anmeldetag: 06.04.2023
(51) Int. Cl.: A61B 50/30, A61B 50/20, A61B 50/00

(54) **STERILVERPACKUNG MIT INTEGRIERTER DÄMPFUNG**
STERILE PACKAGING WITH INTEGRATED DAMPING
EMBALLAGE STÉRILE À AMORTISSEMENT INTÉGRÉ

(30) Priorität: 12.04.2022 DE 102022108827
(43) Veröffentlichungstag der Anmeldung: 24.07.2024
(73) Patentinhaber: Aesculap AG, 78532 Tuttlingen (DE)
(72) Erfinder: SAUTER, Wolfgang, 78603 Renquishausen (DE); ETZEL, Florian, 71364 Winnenden (DE)
(74) Vertreter: Winter, Brandl - Partnerschaft mbB
(86) Internationale Anmeldenummer: PCT/EP2023/059276
(87) Internationale Veröffentlichungsnummer: WO 2023/198633

(56) Entgegenhaltungen:
- WO-A1-95/02548
- WO-A1-98/51585
- WO-A2-2006/028933
- DE-A1- 19 725 499
- US-A- 5 487 470

## Beschreibung

### Technisches Gebiet

Die vorliegende Offenbarung betrifft eine medizinische Sterilverpackung eines medizinischen Produkts.

Medizinische Produkte müssen zur Wahrung ihrer Sterilität sowie zum Schutz vor Beschädigungen während ihres Transports und/oder Lagerung in geeigneter Weise verpackt werden. Hierzu haben sich in der jüngeren Vergangenheit Sterilverpackungen mit wenigstens zwei Sterilbarrieren aus bevorzugt einem Kunststoffmaterial und/oder einer Kunststofffolie sowie einer weiteren Umverpackung beispielsweise aus einem Kartonmaterial durchgesetzt.

### Stand der Technik

Es ist bekannt, medizinische Produkte wie Implantate, Instrumente, Zubehör, insbesondere Schläuche, Behälter, etc., Spritzen und dergleichen medizinische Utensilien in Sterilverpackungen zu lagern, die aus einer inneren, das medizinische Produkt unmittelbar umgebenden Sterilbarriere, einer äußeren, die innere Sterilbarriere lagernden Sterilbarriere und einer die äußere Sterilbarriere aufnehmenden Umverpackung bestehen.

Oftmals ist wenigstens eine der Sterilbarrieren zusätzlich aus einem durchstechsicheren Gewebe gefertigt, um ein nach außen dringen des medizinischen Produkts zu verhindern. Auch werden häufig Sensoreinrichtungen innerhalb der jeweiligen Sterilbarrieren vorgesehen, die das Eindringen von Luft anzeigen und damit die Unsterilität des darin aufgenommenen medizinischen Produkts anzeigen.

Allgemein sind auch stoßabsorbierende Verpackungsmittel wie Kunststofffolien bekannt, die blasen- oder schlauchartige Kompartiments/Taschen ausbilden, in denen Luft eingeschlossen ist und die somit einzelne/separate, über die Kunststofffolie gleichmäßig verteilte Luftpolster ausbilden. Deren Aufgabe ist es, das darin verpackte Produkt gegen äußere Einflüsse wie Vibrationen oder Schläge zu schützen. Diese können beispielsweise durch zwei aufeinander laminierte Kunststofffolien erzeugt werden, die an der entsprechenden Stelle die in sich abgeschlossenen Lufttaschen ausbilden.

Alternativ hierzu ist es auch bekannt, Umverpackungen aus einem mehrlagigen Kartonmaterial mit zwei parallelbeabstandeten Deckplatten und einer wellenartig geformten Zwischenplatte bereitzustellen, die folglich bei einer äußeren Krafteinwirkung deformiert und so stoßabsorbierend wirkt.

Obgleich durch das vorstehend genannte Verpackungsmaterial sowohl Sterilität als auch Aufprallschutz gewährleistet werden kann, haben sich insbesondere hinsichtlich der letztgenannten Eigenschaft sowie insbesondere im Bereich der Medizin Probleme ergeben, wie sie nachfolgend angedeutet sind:
- Bei stoßabsorbierenden Kartonagen ist es notwendig, diese trocken zu lagern, um deren Form, insbesondere der stoßabsorbierenden Zwischendecke aufrecht zu erhalten. Auch sind derartige Materialien in der Regel unelastisch, d.h. sie werden bei äußeren Krafteinflüssen plastisch verformt und verlieren daher die ursprünglichen Dämpfungseigenschaften.
- Stoßabsorbierende Kunststofffolien sind in der Regel Konfektionswaren mit standardisierten Polstertaschengrößen und -abständen. In ungünstigen Fällen können daher darin eingepackte Produkte abschnittsweise an Bereichen zwischen den Polstertaschen vorragen und daher ungeschützte exponierte Produktabschnitte bilden. Daher werden derartige Produkte häufig in mehrere Lagen derartiger stoßabsorbierender Kunststofffolien eingewickelt, um alle ggf. vorragenden Produktabschnitte zu polstern, wodurch jedoch die Gesamtverpackungsschicht erheblich aufträgt.

Aus der WO 2006 / /028 933 A2 ist eine Verpackung bekannt, bei der zwei Folien in einer kofferförmigen Schutzverpackung angeordnet sind. Wenn zwei Hälfen der Schutzverpackung umgeklappt werden, dann liegen die Folien aneinander auf und bilden einen Aufnahmeraum für ein medizinisches Produkt.

Bei allgemeinen Verpackungen ist es bekannt, zu schützende Gegenstände zwischen zwei Luftpolster anzuordnen, um die Gegenstände vor Erschütterungen zu schützen. So zeigen die WO 98 / 51585 A1 und die WO 95 / 02548 A1 jeweils ein aufblasbares Luftkissen, das derart um den Gegenstand gefaltet werden kann, dass der zu schützende Gegenstand von zwei Seiten von dem Luftkissen umgeben ist.

Aus der US 5 487 470 A ist ferner eine Sterilverpackung für medizinische Produkte mit zwei Sterilbarrieren bekannt.

### Kurzbeschreibung der Offenbarung

Ausgehend von dem vorstehend genannten Stand der Technik ist eine Aufgabe der vorliegenden Offenbarung, eine medizinische Sterilverpackung für medizinische Produkte bereitzustellen, welche einen geeigneten Schutz gegen äußere Krafteinflüsse schafft und eine ausreichende Sterilität des darin verpackten Produkts gewährleistet.

Diese Aufgabe wird durch eine medizinische Sterilverpackung mit den Merkmalen des Patentanspruchs 1 gelöst.

Die vorliegende Offenbarung betrifft demzufolge eine medizinische Sterilverpackung zum Verpacken eines medizinischen Produkts mit einer ersten Sterilbarriere, die dafür vorbereitet und ausgebildet ist, einen ersten Aufnahmeraum auszubilden, um das medizinische Produkt in, vorzugsweise vakuumiert, verschweißter/versiegelter Weise darin aufzunehmen, einer zweiten Sterilbarriere, die dafür vorbereitet und ausgebildet ist, einen zweiten Aufnahmeraum zur Aufnahme der ersten Sterilbarriere auszubilden und einer äußeren Schutzverpackung, die dafür vorbereitet und ausgebildet ist, die erste und die zweite Sterilbarriere aufzunehmen. Offenbarungsgemäß weist die zweite Sterilbarriere eine Folie oder eine Doppelmembran auf, die wenigstens zwei Lufttaschen ausbildet. Die zwei Lufttaschen sind durch eine vordefinierte Umklapp- oder Faltkante voneinander getrennt, wodurch im umgeklappten Zustand der Doppelmembran bzw. Folie längs der Umklapp- oder Faltkante die beiden Lufttaschen derart fluchtend aneinander zu liegen kommen, dass dazwischen der zweite Aufnahmeraum erzeugbar ist.

In anderen Worten besteht die vorliegende Offenbarung darin, dass zumindest eine von wenigstens zwei Sterilbarrieren eine Folie aus der Doppelmembran aufweist oder ist. Eine Membran der Folie bildet wenigstens zwei sich auf nur einer Folienseite auswölbende Lufttaschen aus, wohingegen die andere Membran im Wesentlichen planar verbleibt. Die Folie hat ferner eine vordefinierte Umklapp- oder Faltkante, die sich derart zwischen den zwei Lufttaschen erstreckt, dass im umgeklappten Zustand der Folie längs der vordefinierten Umklapp- oder Faltkante die Lufttaschen paarweise im Wesentlichen fluchtend und die planare Membran abschnittsweise im Wesentlichen flächig aneinander zu liegen kommen, sodass zwischen den planaren, flächig aneinander liegenden Membranabschnitten im Bereich der fluchtenden Lufttaschen der verschweiß-/versiegelbare Aufnahmeraum erzeugbar ist, vorzugsweise durch plastische oder elastische Deformation der planaren, flächig aneinander liegenden Membranabschnitte.

In nochmals anderen Worten weist die Sterilverpackung die erste Sterilbarriere, die zweite Sterilbarriere und die äußere Schutzverpackung auf. Die zweite Sterilbarriere ist eine zweilagige Folie. Zwischen den zwei Folien ist Luft eingeschweißt, sodass die Folie Lufttaschen ausbildet. Die Lufttaschen der Folie müssen nicht zwangsläufig symmetrisch ausgebildet sein, sondern können eine ausgebeulte/konkav ausgewölbte und eine planare Seite aufweisen. Die Lufttaschen werden um eine Klappkante umgeklappt, sodass die planaren Seiten jeweils aneinander anliegen bzw. flächig abschließen. Zwischen den planaren Seiten/ Membranabschnitten wird der Aufnahmeraum zur Aufnahme der ersten Sterilbarriere ausgebildet.

Der Kern der vorliegenden Offenbarung besteht demzufolge darin, dass die zumindest zwei Lufttaschen derart um die erste Sterilbarriere umgeklappt werden, dass die Lufttaschen jeweils übereinander angeordnet liegen und die erste Sterilbarriere (vollständig) umschließen/umfassen, um so das zu lagernde Produkt (vollständig) zwischen sich aufzunehmen. An der Kontaktfläche der zwei Lufttaschen wird demnach der Aufnahmeraum für die erste Sterilbarriere gebildet.

Im Wesentlichen planar bedeutet in diesem Zusammenhang, dass die Membran platt ist. Beispielsweise durch Druckschwankungen in der Luftblase kann es aber zu einer geringen konvexen Auswölbung oder auch zu einer ebenso geringen konkaven Einbuchtung der elastischen Membran kommen. Diese geringfügigen Abweichungen von der planaren Membran sind im Sinne der Offenbarung auch als im Wesentlichen planar anzusehen.

Die Umklapp- oder Faltkante kann insbesondere eine Faltlinie ausbilden, die zwei nebeneinanderliegende Gaspolster, insbesondere Lufttaschen / Luftpolster, voneinander trennt. Dabei verbindet die Umklapp- oder Faltkante insbesondere die beiden Membranen der Doppelmembran miteinander. Die Umklapp- oder Faltkante kann dabei entweder durch Punktschweißen oder durch zumindest abschnittsweise linienförmige Schweißnähte gebildet werden. Sollte die Umklapp- oder Faltkante durch Punktschweißen realisiert werden, dann können die nebeneinanderliegenden Luftpolster derart abschnittsweise miteinander verbunden sein, dass die Luft in den einzelnen Polstern in Verbindung stehen kann. Die Schweißnaht kann sich auch über die gesamte Länge der Umklapp- oder Faltkante erstrecken, sodass die einzelnen Luftpolster voneinander unabhängig sind. Ferner können die linienförmigen Schweißnähte unterbrochen sein und eine Verbindung der einzelnen Luftpolster miteinander ermöglichen. Mehrere, beispielsweise zwei, Schweißnähte- oder linien können nebeneinander, insbesondere parallel, angeordnet sein und die Umklapp- oder Faltkante zwischen den Luftpolstern bilden.

Die vorliegende Offenbarung weist die folgenden Vorteile auf:
Da die Sterilverpackung zwei Sterilbarrieren aufweist, ist das medizinische Produkt in der Sterilverpackung doppelt steril geschützt.

Das medizinische Produkt wird durch die Lufttaschen vor Einflüssen von außen wie Erschütterungen oder Vibrationen geschützt.

Da die Lufttaschen in die Sterilbarriere integriert sind, müssen keine zusätzlichen Luftpolster oder andere Dämpfungsmittel in die äußere Schutzverpackung eingebracht werden. Dadurch entfallen Teile und zusätzliche Arbeitsschritte beim Verpacken.

Schließlich bilden die beiden Lufttaschen in zusammengeklapptem Zustand der Folie einen Zwischenspalt, in den das in die erste Sterilbarriere bereits eingeschweißte Produkt vollständig (unter elastischer Verformung der zweiten Sterilbarriere) einlegbar ist, wodurch diese vollständig vom (einlagigen) Luftpolster umgeben wird. Damit kann das Produkt optimal geschützt und so viel wie möglich an Verpackungsmaterial eingespart werden.

Vorzugsweise weist die zweite Sterilbarriere zwei Lufttaschen auf, die durch eine oder mehrere vordefinierte Umklapp- oder Faltkanten voneinander getrennt sind und derart umklappbar sind, dass eine weitere Tasche zum Aufnehmen der ersten Sterilbarriere zwischen den Lufttaschen gelagert ist. Die erste Sterilbarriere mit dem medizinischen Produkt wird in die weitere Tasche der zweiten Sterilbarriere eingebracht und darin, vorzugsweise vakuumiert, verschweißt. Die zweite Sterilbarriere kann zusätzlich zu der weiteren Tasche die zwei Lufttaschen aufweisen, die von der weiteren Tasche durch die zumindest eine oder mehrere vordefinierten Umklapp- oder Faltkanten getrennt sind. Diese Ausgestaltung ist sehr einfach zu fertigen.

In anderen Worten ausgedrückt hat die zweite Sterilbarriere zwei als Lufttaschen ausgebildete Abschnitte und einen als weitere Aufnahmetasche ausgebildeten Abschnitt, die über Umklapp- oder Faltenkanten zu einem einzigen Bauteil einstückig zusammengesetzt sind. Die weitere Aufnahmetasche dient zur Aufnahme der ersten Sterilbarriere und damit des darin befindlichen medizinischen Produkts. Dadurch wird quasi zwangsläufig die Relativlage des medizinischen Produkts zu den beiden Lufttaschen festgelegt, sobald das medizinische Produkt samt dessen dieses umgebende erste Sterilbarriere in der Aufnahmetasche platziert und ggf. durch Vakumieren der Aufnahmetasche fixiert ist. Werden die Lufttaschen nunmehr längs der Umklapp- oder Faltenkanten umgelegt, kommt das medizinische Produkt automatisch in korrekter Ausrichtung zwischen den Lufttaschen zu liegen.

**In** einer vorteilhaften Gestaltung können die Lufttaschen derart um die weitere (Aufnahme-)Tasche umgeklappt werden, dass die weitere Tasche von zumindest zwei Seiten von den Lufttaschen umgeben ist. Die Lufttaschen sind zumindest teilweise komprimierbar. D.h., wenn die weitere Tasche mit der ersten Sterilbarriere zwischen den zwei Lufttaschen anliegt, drücken sich die Lufttaschen (teilweise) ein und bilden den Aufnahmeraum für die weitere Tasche bzw. das medizinische Produkt samt dessen erster Sterilbarriere in der weiteren Tasche. Dadurch ist das medizinische Produkt gedämpft gelagert und von den Lufttaschen geschützt.

Nach einem weiteren optionalen Merkmal der Offenbarung sind die beiden Lufttaschen jeweils auf gegenüberliegenden Seiten der weiteren Tasche angeordnet. D.h. auf jeder Seite der weiteren Tasche ist eine Lufttasche angeordnet und jeweils über Umklapp- oder Faltkanten mit der weiteren (Aufnahme-) Tasche stoffeinstückig verbunden. Die Lufttaschen und die weitere Tasche sind demnach jeweils durch die Umklapp- oder Faltkanten scharnierartig räumlich getrennt bzw. beabstandet. In dieser Ausgestaltung wird jede der Lufttaschen jeweils um die Umklapp- oder Faltkanten auf eine gegenüberliegende Seite der weiteren Tasche (Z-förmig) geklappt. So wird die weitere Tasche durch die Lufttaschen umschlossen.

Alternativ können die beiden Lufttaschen auf der gleichen Seite von der weiteren Tasche mit der ersten Sterilbarriere angeordnet sein. D.h. auf einer Seite der Tasche sind die Lufttaschen nebeneinander angeordnet. Die Lufttaschen sind untereinander durch die Umklapp- oder Faltkanten getrennt. Die Lufttaschen sind von der weiteren Tasche ebenfalls durch die Umklapp- oder Faltkante getrennt. Beim Umklappen der Lufttaschen um die weitere Tasche wird zuerst die Lufttasche, die neben der weiteren Tasche anliegt, an die Tasche geklappt. Anschließend wird die weitere Lufttasche auf die Seite der weiteren Tasche geklappt, die der ersten Lufttasche gegenüberliegt.

Nach einem weiteren optionalen Merkmal der Offenbarung ist die Folie der zweiten Sterilbarriere aus einer Doppelmembran (gefertigt), wobei sich eine Membran konkav nach außen wölbt und eine Membran planar verbleibt. In anderen Worten besteht die Folie aus zwei Folien. Eine erste Folie bildet die konkaven Taschen aus, während eine zweite Folie planar ist und die Taschen abschließt. Die konkave Folienseite ermöglicht ausreichend Volumen für die Luft innerhalb der Lufttaschen. Die planare Membran ermöglicht, dass die planaren Membranabschnitte nach dem Umklappen möglichst flächig aneinander anliegen.

Nach einem weiteren optionalen Merkmal der Offenbarung kommt beim Umklappen der Folie die planare Membran abschnittsweise im Wesentlichen flächig aneinander zu liegen. Die flächig aneinander anliegenden planaren Membranabschnitte bilden den Aufnahmeraum für die erste Sterilbarriere aus. Dadurch umschließen die planaren Membranabschnitte die erste Sterilbarriere möglichst vollständig. Durch das Umschließen der ersten Sterilbarriere mit der Membran und anschließendem Versiegeln der planaren Membranabschnitte wird die zweite Sterilbarriere gebildet.

Nach einem weiteren optionalen Merkmal der Offenbarung ist der verschweiß- /versiegelbare Aufnahmeraum zwischen den planaren, flächig aneinander liegenden Membranabschnitten im Bereich der fluchtenden Lufttaschen erzeugbar. Der Aufnahmeraum wird vorzugsweise durch plastische oder elastische Deformation der planaren, flächig aneinander liegenden Membranabschnitte erzeugt. Die elastische Membran verformt sich und nimmt die Form der ersten Sterilbarriere an. Da die Membran die Form der ersten Sterilbarriere annimmt, wird die erste Sterilbarriere weitestgehend umschlossen. Das bewirkt eine gute Dämpfung mit möglichst wenig Bewegung und eine sterile Verpackung.

Nach einem weiteren optionalen Merkmal der Offenbarung ist die zweite Sterilbarriere eine Folie mit einer Doppelmembran. Eine erste konvexe Membran der Doppelmembran ist aus einem festen Material gefertigt und eine zweite, planare Membran ist elastisch. Die erste Membran ist relativ fest, damit sie tiefgezogen werden kann. Durch das Tiefziehen werden die Lufttaschen gebildet. Ferner fungiert die feste Membran als ein Durchstechschutz, der verhindert, dass ein spitzes Instrument durch die Sterilverpackung durchsticht. Die zweite Membran ist elastisch, damit sie die Form der ersten Sterilbarriere annehmen kann und die erste Sterilbarriere weitestgehend umfasst/ umschließt.

Nach einem weiteren optionalen Merkmal der Offenbarung weist die erste Sterilbarriere zwei Folien auf und ist dafür vorbereitet und ausgebildet, das medizinische Produkt derart zwischen den zwei Folien zu verschweißen/versiegeln, dass eine Relativbewegung zwischen den Folien und dem medizinischen Produkt vermindert ist. Das medizinische Produkt wird also so fest zwischen den zwei Folien verschweißt, dass es sich nicht in den Folien bewegen/ verrutschen kann. Durch die verminderte Relativbewegung zwischen den Folien und dem medizinischen Produkt wird eine Partikelentstehung durch Reibung zwischen den Folien und dem medizinischen Produkt ausgeschlossen oder zumindest vermindert. Partikel können sterile medizinische Produkte verunreinigen. Eine wiederholte Säuberung und Sterilisation wäre im schlimmsten Fall die Folge. Die fest verschweißten Folien sind also eine Sterilbarriere und ermöglichen zusätzlich einen effektiven Partikel- und Abriebschutz.

Nach einem weiteren optionalen Merkmal der Offenbarung ist die erste Sterilbarriere steril in der zweiten Sterilbarriere gelagert. Um die Funktionalität einer Doppel-Sterilbarriere bzw. einer doppelten Sterilität zu ermöglichen, muss die erste Sterilbarriere in eine weitere sterile Verpackung/ zweite Sterilbarriere eingebracht werden. Das ist die zweite Sterilbarriere. Da die zweite Sterilbarriere die erste Sterilbarriere umfasst und verschweißt ist, funktioniert sie als Sterilbarriere.

Nach einem weiteren optionalen Merkmal der Offenbarung weist die Sterilverpackung mehrere Versiegelungen auf. Eine erste und eine zweite Versiegelung versiegeln die erste Sterilbarriere, eine dritte, eine vierte und eine fünfte Versiegelung versiegeln jeweils die Doppelmembran neben den Lufttaschen der zweiten Sterilbarriere. Eine sechste Versiegelung versiegelt die zwei aufeinanderliegenden Lufttaschen miteinander.

Die erste Versiegelung und die zweite Versiegelung versiegeln die beiden Folien der ersten Sterilverpackung an beiden Seiten des medizinischen Produkts. Drei Versiegelungen halten die zwei Membranen der Doppelmembran der zweiten Sterilbarriere zusammen. Die zwei Membranen können auch zwei Folien sein, die durch die Versiegelungen miteinander verschweißt sind. Die drei Versiegelungen sind an den beiden äußeren Seiten der Lufttaschen und zwischen den Lufttaschen angebracht. Die letzte Versiegelung versiegelt die beiden umgeklappten, aneinander aufliegenden Lufttaschen miteinander, um die zweite Sterilbarriere um die erste Sterilbarriere zu bilden. Durch die Versiegelungen wird die Sterilität der einzelnen Verpackungsschichten bzw. Barrieren gesichert.

Nach einem weiteren optionalen Merkmal der Offenbarung weist die zweite Sterilbarriere genau zwei Lufttaschen auf. Durch zwei Lufttaschen kann genau ein medizinisches Produkt zwischen den zwei Lufttaschen gedämpft gelagert werden. Es ist selbstverständlich auch denkbar, dass die zweite Sterilbarriere mehrere Lufttaschen aufweist. Dadurch können mehrere medizinische Produkte gedämpft gelagert werden.

Die Aufgabe der vorliegenden Offenbarung kann ferner durch ein Verfahren zur Herstellung einer Sterilverpackung gemäß einem der vorstehenden Aspekte gelöst werden. Das offenbarungsgemäße Verfahren weist die folgenden Schritte auf: Die erste Sterilbarriere wird durch Versiegeln zweier Folien (um das medizinische Produkt herum) hergestellt. Die zweite Sterilbarriere wird beispielsweise durch Tiefziehen und anschließendes Versiegeln der Lufttaschen hergestellt. Die erste Sterilbarriere wird auf einen der planaren Membranabschnitte der zweiten Sterilbarriere aufgelegt oder in eine Aufnahmetasche zwischen den beiden Lufttaschen eingesteckt, welche zusammen mit den beiden Lufttaschen zu einem einzigen Bauteil zusammengefügt und mittels Faltenlinien voneinander räumlich beabstandet werden. Nach dem Auflegen/ Einlegen der ersten Sterilbarriere wird eine der Lufttaschen derart umgeklappt, dass die Lufttaschen die erste Sterilbarriere bzw. die Aufnahmetasche für die erste Sterilbarriere umschließen. Die planaren Membranabschnitte bilden den Aufnahmeraum um die erste Sterilbarriere/Aufnahmetasche. Die Lufttaschen werden miteinander versiegelt/ verschweißt, um die zweite Sterilbarriere um die erste Sterilbarriere/ Aufnahmetasche herum zu schaffen. Die zweite Sterilbarriere mitsamt der ersten Sterilbarriere wird in die äußere Schutzverpackung eingelegt.

Das offenbarungsgemäße Verfahren hat die folgenden Vorteile. Die Dämpfung für die erste Sterilbarriere ist in der zweiten Sterilbarriere integriert. Somit muss keine zusätzliche Luftpolsterfolie oder ähnliche Dämpfungsmittel in die äußere Schutzverpackung eingebracht werden. Das Verfahren stellt ferner sehr einfach und kostengünstig eine doppelte Sterilbarriere bereit.

Es kann auch vorteilhaft sein, die zweite Sterilbarriere dadurch herzustellen, dass die erste Sterilbarriere in der weiteren Tasche der zweiten Sterilbarriere steril verschweißt wird und anschließend die Lufttaschen oder Luftpolster der zweiten Sterilbarriere um die weitere Tasche umgeklappt werden. Somit sind die Lufttaschen und die sterile Aufnahmetasche einfach zu fertigen.

Das Verfahren kann ferner die folgenden Schritte aufweisen: Das medizinische Produkt wird auf eine erste Folie aufgelegt. Eine zweite Folie wird auf die erste Folie mit dem medizinischen Produkt aufgelegt. Danach werden die beiden Folien jeweils an beiden Seiten des medizinischen Produkts vakuumiert versiegelt/verschweißt. Durch das Verschweißen des medizinischen Produkts zwischen den Folien entsteht eine Sterilbarriere. Das Produkt kann sich innerhalb der Folien nicht oder nur minimal bewegen. Das verhindert den Abrieb von Partikeln durch Reibung.

Ferner können in dem offenbarungsgemäßen Verfahren zur Herstellung der zweiten Sterilbarriere die folgenden Schritte durchgeführt werden:
- Tiefziehen einer ersten äußeren Membran, um die Lufttaschen zu bilden, die nebeneinanderliegen und in dieselbe Richtung geöffnet sind;
- Versiegeln der zwei nebeneinanderliegenden Lufttaschen an den jeweiligen Öffnungen durch eine zweite innere Membran, um nebeneinanderliegende Lufttaschen mit einer gewölbten und einer planaren Seite zu bilden;
- Nach dem Einlegen der ersten Sterilbarriere, Umklappen der Lufttaschen, sodass die Lufttaschen im Wesentlichen fluchtend und die planaren Seiten flächig aneinander anliegen und einen Aufnahmeraum zur Aufnahme der ersten Sterilbarriere ausbilden; und
- Versiegeln der aneinander liegenden Lufttaschen miteinander.

Die erste Membran/Folie wird tiefgezogen, um offene Taschen/konkave Ausbeulungen herzustellen. Diese offenen Taschen werden von der zweiten Membran/Folie abgeschlossen. Dazu werden die beiden Membranen miteinander versiegelt. Die zweite Sterilbarriere weist nun die konkav ausgewölbte Membran und die planare Membran auf, die zusammen die Lufttaschen bilden. Die erste Sterilbarriere wird auf der planaren Membran der zweiten Sterilbarriere aufgelegt. Anschließend wird eine der Lufttaschen um die Umklapp- oder Faltkante umgeklappt. Die Lufttaschen werden derart umgeklappt, dass die Lufttaschen im Wesentlichen fluchtend und die planaren Seiten flächig aneinander anliegen. Die planaren Seiten bilden den Aufnahmeraum für die erste Sterilbarriere aus. Dazu passen sich die elastischen, planaren Membranabschnitt an die Form der ersten Sterilbarriere an. Durch das Verfahren wird einfach und kostengünstig die zweite Sterilbarriere mit integrierten Lufttaschen bereitgestellt.

Die vorliegende Offenbarung betrifft ferner ein System aus einer medizinischen Sterilverpackung nach einem der vorstehenden Aspekte und einem medizinischen Produkt, das in der medizinischen Sterilverpackung steril verpackt ist. Die wenigstens zwei Lufttaschen der zweiten Sterilbarriere sind derart um das medizinische Produkt umgeklappt, dass eine planare Membran der zweiten Sterilbarriere in Richtung des medizinischen Produkts zeigt und der Aufnahmeraum zur Aufnahme der ersten Sterilbarriere in der planaren Membran ausgebildet ist. Die elastische, planare Membran nimmt die Form der ersten Sterilbarriere an und umfasst die erste Sterilbarriere vollständig. Dadurch wird sichergestellt, dass sich die erste Sterilbarriere nicht in der zweiten Sterilbarriere bewegen kann und auch steril in der zweiten Sterilbarriere gelagert ist. Die andere konvexe Membran ist fest und könnte sich nicht an die Form der ersten Sterilbarriere anpassen.

Nach einem weiteren optionalen Merkmal der Offenbarung ist die Ausdehnung der planaren Membran größer als das medizinische Produkt. Der Aufnahmeraum der planaren Membran umschließt das medizinische Produkt vollständig oder annähernd vollständig. Da der planare Membranabschnitt, der die erste Sterilbarriere umfasst, größer ist als das medizinische Produkt und damit auch größer als die erste Sterilbarriere mit dem medizinischen Produkt, kann der planare Membranabschnitt die erste Sterilbarriere vollständig umfassen/ umgreifen.

### Kurzbeschreibung der Figuren

Fig. 1 zeigt einen Längsschnitt durch eine Sterilverpackung gemäß einer ersten Ausführungsform der vorliegenden Offenbarung mit einer ersten Sterilbarriere und einer zweiten Sterilbarriere;
Fig. 2 zeigt einen Längsschnitt durch die erste Sterilbarriere mit einem medizinischen Produkt;
Fig. 3 zeigt einen Längsschnitt durch die zweite Sterilbarriere;
Fig. 4 zeigt ein Ablaufdiagramm eines offenbarungsgemäßen Verfahrens zur Herstellung der Sterilverpackung gemäß der vorliegenden Offenbarung;
Fig. 5 zeigt die zweite Sterilbarriere während der Herstellung nach dem offenbarungsgemäßen Verfahren; und
Fig. 6 zeigt die zweite Sterilbarriere in fertigem Zustand;
Fig. 7 zeigt eine Sterilverpackung gemäß einer zweiten Ausführungsform der vorliegenden Offenbarung; und
Fig. 8 zeigt eine Sterilverpackung gemäß einer dritten Ausführungsform der vorliegenden Offenbarung.

### Detaillierte Beschreibung der Figuren

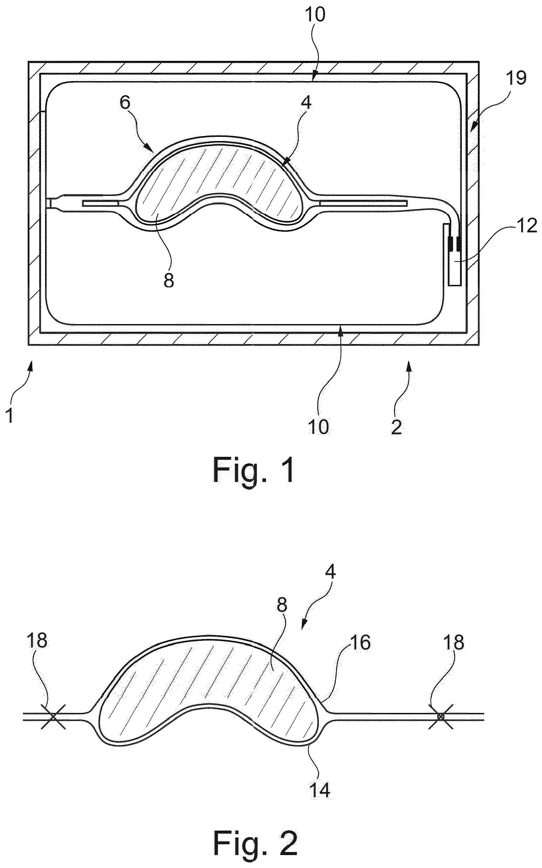

Fig. 1 zeigt einen Längsschnitt durch eine Sterilverpackung 1. Die Sterilverpackung 1 weist eine Schutzverpackung 2 auf, die vorzugsweise ein Karton ist. Die Schutzverpackung 2 ist annähernd rechteckig. Innerhalb der Schutzverpackung 2 sind zwei Sterilbarrieren angeordnet. Eine erste Sterilbarriere 4 ist dabei steril innerhalb einer zweiten Sterilbarriere 6 angeordnet. Ein medizinisches Produkt 8 ist innerhalb der ersten Sterilbarriere 4 versiegelt bzw. verschweißt ist. Die zweite Sterilbarriere 6 umgibt die erste Sterilbarriere 4 und steht an seiner Außenseite mit der Schutzverpackung 2 in Kontakt. D.h. die erste Sterilbarriere 4 ist von der zweiten Sterilbarriere 6 komplett umschlossen. Die zweite Sterilbarriere 6 weist zwei längliche Lufttaschen 10 auf. Jeweils eine der zwei Lufttaschen 10 füllt annähernd eine Hälfte des Volumens der Schutzverpackung 2 aus. Somit teilen die zwei Lufttaschen 10 die Schutzverpackung 2 in zwei Hälften. Zwischen den zwei Lufttaschen 10 ist die erste Sterilbarriere 4 gelagert. Die zwei Lufttaschen 10 sind an der einen Seite zusammengeschweißt. An der anderen Seite weisen die zwei Lufttaschen 10 jeweils einen Verschweiß-Zipfel 12 auf. Die beiden Verschweiß-Zipfel 12 sind zusammengeschweißt. So umgeben die zwei Lufttaschen 10 die erste Sterilbarriere 4 vollständig.

Die zwei Lufttaschen 10 lagern die erste Sterilbarriere 4 dämpfend. Das heißt die zwei Lufttaschen 10 schützen die Sterilbarriere 4 vor Erschütterungen. Zusätzlich stabilisieren die zwei Lufttaschen 10 die Schutzverpackung 2. Durch die versiegelten Verschweiß-Zipfel 12, ist die erste Sterilbarriere 4 steril innerhalb der zweiten Sterilbarriere 6 gelagert.

Fig. 2 zeigt einen Längsschnitt durch die erste Sterilbarriere 4. Die erste Sterilbarriere 4 weist zwei Folien auf, nämlich eine Unterfolie 14 und eine Oberfolie 16. Das medizinische Produkt 8 liegt auf der Unterfolie 14 auf. Die Oberfolie 16 wird auf die Unterfolie 14 mit dem medizinischen Produkt 8 gelegt und vakuumiert mit der Unterfolie zusammengeschweißt. Die Unterfolie 14 und die Oberfolie 16 werden dabei jeweils auf beiden Seiten des medizinischen Produkts 8 zusammengeschweißt. Dadurch entstehen zwei Schweißpunkte/ Versiegelungen 18.

Durch das vakuumierte Einschweißen des medizinischen Produkts 8 in die Unterfolie 14 und die Oberfolie 16 entstehen selbst bei einer großen Transportbelastung der Sterilverpackung 1 keine oder nur minimale Relativbewegungen zwischen dem medizinischen Produkt 8 und den beiden Folien 14, 16. Dadurch kann eine Entstehung von Partikeln durch Abrieb ausgeschlossen bzw. zumindest vermindert werden. Die erste Sterilbarriere 4 ist also gleichzeitig ein Partikel- und ein Abriebschutz.

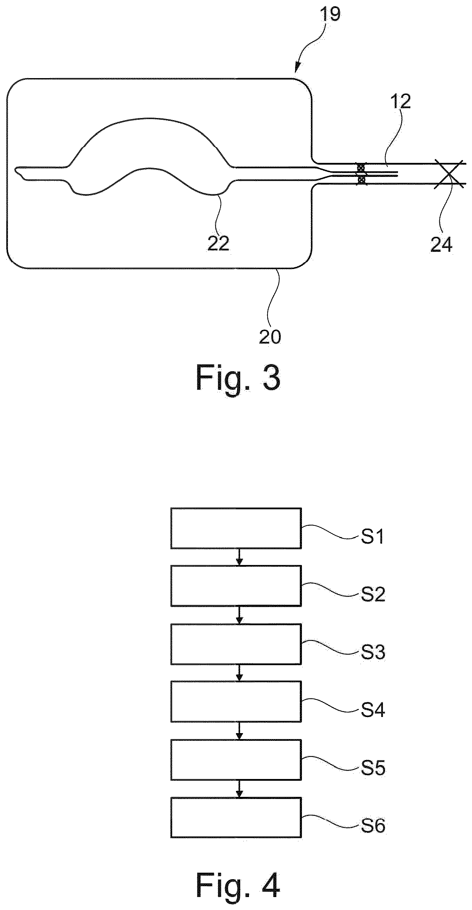

Fig. 3 zeigt einen Längsschnitt durch die zweite Sterilbarriere 6. Die erste Sterilbarriere 4 ist zwischen den zwei Lufttaschen 10 der zweiten Sterilbarriere 6 eingebettet. Die zweite Sterilbarriere 6 ist eine oder ist aus einer Folie, die eine Doppelmembran 19 ist. Die Doppelmembran 19 weist eine erste (äußere) Membran 20 und eine zweite (innere) Membran 22 auf. Die (äußere) Membran 20 steht in Kontakt zu der Schutzverpackung 2 und ist fest. D.h. die (äußere) Membran 20 ist nicht elastisch bzw. nicht ohne Weiteres verformbar. Die (innere) Membran 22 steht in Kontakt mit der ersten Sterilbarriere 4 und ist elastisch. D.h. die ersten Sterilbarriere 4 sinkt in die (innere) Membran 22 ein, die Membran 22 passt sich an die Form der ersten Sterilbarriere 4 an und umschließt diese (fast) komplett. Die (innere) Membran 22 bildet einen Aufnahmeraum für die erste Sterilbarriere 4 aus. Die zwei Lufttaschen 10 sind jeweils luftdicht versiegelt. Die zwei Verschweiß-Zipfel 12 der zwei Lufttaschen 10 sind miteinander verschweißt/versiegelt und bilden dadurch einen gemeinsamen Verschweiß-Zipfel der zweiten Sterilbarriere 6 aus. Die beiden Verschweiß-Zipfel 12 sind mit einer Versiegelung 24 miteinander verschweißt/versiegelt.

Fig. 4 zeigt ein Ablaufdiagramm von einem Verfahren zur Herstellung der Sterilverpackung 1. In einem ersten Schritt S1 wird die erste Sterilbarriere 4 durch Versiegeln des medizinischen Produkts 8 zwischen zwei Folien 14, 16 hergestellt. Dabei wird das medizinische Produkt 8 auf die Unterfolie 14 gelegt, mit der Oberfolie 16 bedeckt und die beiden Folien 14, 16 vakuumiert verschweißt. In einem weiteren Schritt S2 werden in der festen (äußeren) Membran 20 der zweiten Sterilbarriere 6 durch Tiefziehen zwei Taschen 26 gebildet. Die Taschen 26 liegen nebeneinander und sind beide in dieselbe Richtung geöffnet. Die zwei nebeneinanderliegenden Taschen 26 werden mit der elastischen (inneren) Membran 22 luftdicht abgeschlossen. Die elastische (innere) Membran 22 wird dabei durch drei Versiegelungen 28 versiegelt, jeweils einmal außerhalb der Taschen 26 und einmal zwischen den Taschen 26. Durch das Versiegeln der beiden Membranen 20, 22 werden die zwei Lufttaschen 10 mit den Verschweiß-Zipfeln 12 ausgebildet. Eine Umklapp- oder Faltkante 30 ist zwischen den nebeneinanderliegenden Lufttaschen 10 positioniert. In einem weiteren Schritt S3 wird die erste Sterilbarriere 4 auf die elastische (innere) Membran 22 von einer der Lufttaschen 10 aufgelegt. Die elastische (innere) Membran 22 gibt dabei nach und passt sich der Form der ersten Sterilbarriere 4 an. Die Lufttasche 10, auf der die erste Sterilbarriere 4 nicht liegt, wird in einem weiteren Schritt S4 um die Umklapp- oder Faltkante 30 umgeklappt, wobei die Versiegelung zwischen den zwei Lufttaschen 10 die Umklapp- oder Faltkante 30 ist. Eine der Lufttaschen 10 wird dabei derart umgeklappt, dass eine obere Lufttasche auf der unteren Lufttasche aufliegt, sich die elastischen (innere) Membranabschnitte der Lufttaschen 10 berühren und die erste Sterilbarriere 4 umgeben. Die elastische (innere) Membran 22 bildet dabei den Aufnahmeraum für die erste Sterilbarriere 4 aus. Die beiden Verschweiß-Zipfel 12 werden im Schritt S5 miteinander verschweißt/versiegelt. Die umgeklappte und versiegelte zweite Sterilbarriere 6 mitsamt der ersten Sterilbarriere 4 wird in einem letzten Schritt S6 in die Schutzverpackung 2 eingelegt und die Schutzverpackung 2 verschlossen.

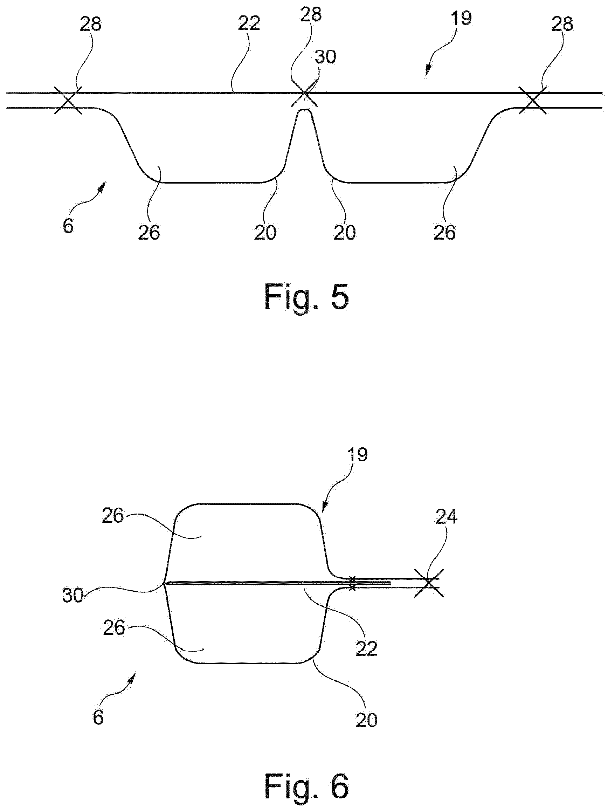

Fig. 5 zeigt die Doppelmembran 19 der zweiten Sterilbarriere 6 nach dem Schritt S2. Dabei sind die beiden Taschen 26 der Unterfolie 20 ausgebildet. Die elastische (innere) Membran 22 ist auf die Öffnungen der Taschen 26 geschweißt, um die Lufttaschen 10 zu bilden. Dazu sind die drei Schweißpunkte/ Versiegelungen 28 ausgebildet. Zwischen den beiden Taschen 26 ist die Umklapp- oder Faltkante 30 ausgebildet.

Fig. 6 zeigt die Doppelmembran 19 der zweiten Sterilbarriere 6 nach der Herstellung. Dabei sind die zwei Taschen 26 aufeinander geklappt und die zwei Verschweiß-Zipfel 12 sind miteinander verschweißt. Die zweite Sterilbarriere 6 weist deshalb den Schweißpunkt/ die Versiegelung 24 auf.

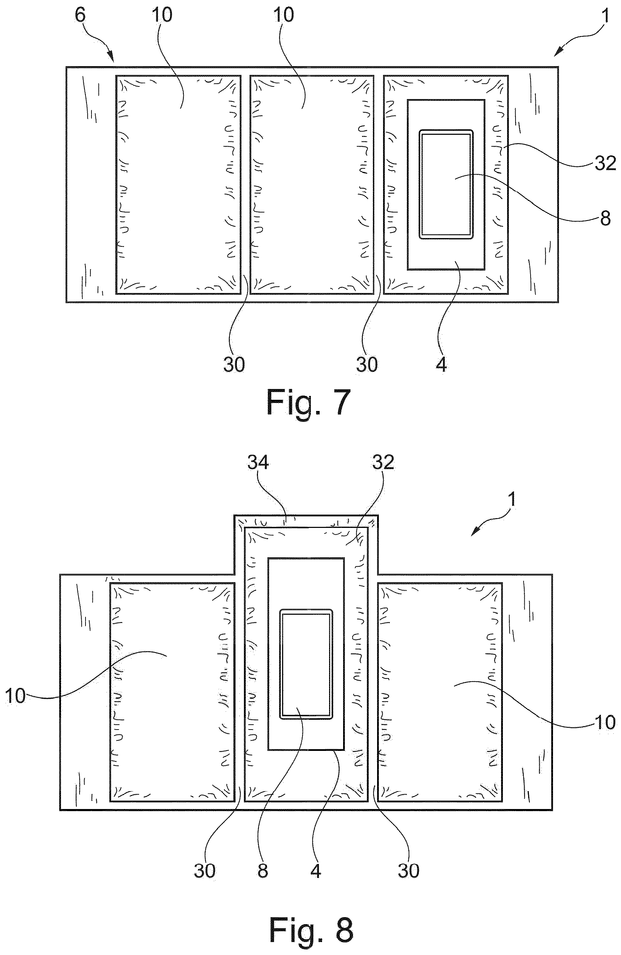

Fig. 7 zeigt eine Sterilverpackung 1 gemäß einer zweiten Ausführungsform in einem ausgeklappten Zustand. Die Sterilverpackung 1 ist demzufolge ein integrales Bauteil bestehend aus der zweiten Sterilbarriere 6 mit den zwei (länglichen/rechteckigen) Lufttaschen 10 und einer zusätzlichen Aufnahmetasche 32 zur Aufnahme eines medizinischen Produkts, das wiederum in der ersten Sterilbarriere verpackt ist. Die zwei Lufttaschen 10 sind in diesem Beispiel unmittelbar nebeneinander positioniert und voneinander durch die (eine) Umklapp- oder Faltkante 30 getrennt. Die zweite Sterilbarriere 6 weist die weitere Tasche 32 auf, die dafür vorbereitet und ausgebildet ist, die erste Sterilbarriere 4 und somit das darin verpackte Produkt aufzunehmen. Die weitere Tasche 32 ist durch eine weitere Umklapp- oder Faltkante 30 von der einen Lufttasche 10 getrennt, die unmittelbar neben der Tasche 32 positioniert ist. Das medizinische Produkt 8 ist in die Tasche 32 eingebracht und wird vorzugsweise in dieser, besonders bevorzugt vakuumiert, eingeschweißt/verschweißt. Die Lufttaschen 10 werden derart (fortlaufend) um die Tasche 32 mit der ersten Sterilbarriere 4 umgeklappt/umgeschlagen, dass an jeder Seite der Tasche 32 eine Lufttasche 10 anliegt. Die Lufttaschen 10 sind teilweise komprimierbar und bilden zwischen sich den Aufnahmeraum für die Tasche 32 mit der ersten Sterilbarriere 4 aus. Die Umklapp- oder Faltkanten 30, die die zwei Lufttaschen 10 und die weitere Tasche 32 trennen, können dabei als linienförmige und/oder punktförmige Schweißnaht ausgebildet sein. Die die linienförmige Schweißnaht kann sich über die gesamte Kantenlänge erstrecken, kann aber auch unterbrochen sein. Sollte die Schweißnaht unterbrochen linienförmig oder punktförmig ausgeführt sein, können die Lufttaschen miteinander in (Fluid- )Verbindung stehen. Die Umklapp- oder Faltkanten 30 können auch aus mehreren parallel Schweißnähten bestehen.

Die Lufttasche 10, die direkt neben der Tasche 32 positioniert ist, wird zuerst auf eine Seite der Tasche 32 geklappt. Anschließend wird die weitere Lufttasche 10 noch einmal umgeklappt, um auf der gegenüberliegenden Seite der Tasche 32 anzuliegen. Dadurch ist die Tasche 32 von beiden Seiten von den Lufttaschen umgeben und geschützt.

Fig. 8 zeigt eine Sterilverpackung 1 gemäß einer dritten Ausführungsform in einem ausgeklappten Zustand. Die beiden Lufttaschen 10 sind in dieser Ausführungsform auf zwei gegenüberliegenden Seiten der Tasche 32 mit der darin befindlichen ersten Sterilbarriere 4 positioniert. Die Tasche 32 ist also zwischen den beiden Lufttaschen 10 angeordnet. Die Tasche 32 ist von den Lufttaschen jeweils durch die Umklapp- oder Faltkante 30 getrennt. Die Tasche 32 weist eine Lasche 34 zum Verschweißen der Tasche 32 auf, nachdem die erste Sterilbarriere 4 mit dem medizinischen Produkt 8 eingelegt wurde. Die beiden Lufttaschen werden jeweils derart an der Umklapp- oder Faltkante 30 umgeklappt, dass eine Lufttasche an jeder Seite der Tasche 32 anliegt.

Dadurch liegt die Tasche und das medizinische Produkt 8 in der Tasche in dem Aufnahmeraum zwischen den Lufttaschen 10 an und wird von (mindestens) zwei Seiten geschützt.

### Bezugszeichenliste

- 1: Sterilverpackung
- 2: äußere Schutzverpackung
- 4: erste Sterilbarriere
- 6: zweite Sterilbarriere
- 8: medizinisches Produkt
- 10: Lufttasche
- 18: Versiegelung
- 19: Doppelmembran
- 20: (äußere) Membran
- 22: (innere) Membran

- 24: Versiegelung
- 28: Versiegelung
- 30: Umklapp- oder Faltkante
- 32: weitere Tasche
- 34: Lasche

## Patentansprüche

1. Medizinische Sterilverpackung (1) zum Verpacken eines medizinischen Produkts (8) aufweisend:
- eine erste Sterilbarriere (4), die dafür vorbereitet und ausgebildet ist, das medizinische Produkt (8) in verschweißter/versiegelter Weise aufzunehmen,
- eine zweite Sterilbarriere (6), die dafür vorbereitet und ausgebildet ist, einen Aufnahmeraum zur Aufnahme der ersten Sterilbarriere (4) in verschweißter/ versiegelter Weise auszubilden, und
- eine äußere Schutzverpackung (2), die dafür vorbereitet und ausgebildet ist, die erste Sterilbarriere (4) und die zweite Sterilbarriere (6) aufzunehmen,
wobei die zweite Sterilbarriere (6) eine, wenigstens zwei Lufttaschen (10) ausbildende Doppelmembran (19) hat oder ist, die durch eine vordefinierte Umklapp- oder Faltkante (30) in der Doppelmembran (19) voneinander getrennt sind, wodurch im umgeklappten Zustand der Doppelmembran (19) längs der Umklapp- oder Faltkante (30) die beiden Lufttaschen (10) derart fluchtend aufeinander zu liegen kommen, dass dazwischen der Aufnahmeraum erzeugbar ist.

2. Medizinische Sterilverpackung (1) nach Anspruch 1, **dadurch gekennzeichnet, dass** die Doppelmembran äußere Membran (20) aufweist, die sich konkav auswölbt und eine innere Membran (22) der Doppelmembran (19) planar verbleibt.

3. Medizinische Sterilverpackung (1) nach Anspruch 1 oder 2, **dadurch gekennzeichnet, dass** beim Umklappen der Lufttaschen (10) die planare Membran (22) abschnittsweise im Wesentlichen flächig aneinander zu liegen kommt.

4. Medizinische Sterilverpackung (1) nach Anspruch 2 oder 3, **dadurch gekennzeichnet, dass** der verschweiß-/versiegelbare Aufnahmeraum zwischen den planaren, flächig aneinander liegenden Membranabschnitten (22) im Bereich der fluchtenden Lufttaschen (10) erzeugbar ist, vorzugsweise durch plastische oder elastische Deformation der planaren, flächig aneinander liegenden Membranabschnitte (22).

5. Medizinische Sterilverpackung (1) nach einem der Ansprüche 1 bis 4, **dadurch gekennzeichnet, dass** die zweite Sterilbarriere (6) genau zwei Lufttaschen (10) aufweist.

6. Medizinische Sterilverpackung (1) nach einem der Ansprüche 1 bis 5, **dadurch gekennzeichnet, dass** die erste konvexe Membran (20) der zweiten Sterilbarriere (6) aus einem festen Material gefertigt ist und die zweite, planare Membran (22) elastisch ist.

7. Medizinische Sterilverpackung (1) nach einem der Ansprüche 1 bis 6, **dadurch gekennzeichnet, dass** die erste Sterilbarriere (4) zwei Folien (14, 16) aufweist und dafür vorbereitet und ausgebildet ist, das medizinische Produkt (8) derart zwischen den zwei Folien (14, 16) zu verschweißen/versiegeln, dass eine Relativbewegung zwischen den Folien (14, 16) und dem medizinischen Produkt (8) vermindert ist.

8. Medizinische Sterilverpackung (1) nach einem der Ansprüche 1 bis 7, **gekennzeichnet durch** mehrere Versiegelungen, wobei eine erste und eine zweite Versiegelung (18) die erste Sterilbarriere (4) versiegeln, eine dritte, eine vierte und eine fünfte Versiegelung (28) jeweils die Doppelmembran (19) neben den Lufttaschen (10) der zweiten Sterilbarriere (6) versiegeln und eine sechste Versiegelung (24) die zwei aufeinanderliegenden Lufttaschen (10) miteinander versiegeln.

9. Medizinische Sterilverpackung (1) nach einem der Ansprüche 1 bis 8, **dadurch gekennzeichnet, dass** die zweite Sterilbarriere (6) zwei Lufttaschen (10) aufweist, die durch vordefinierte Umklapp- oder Faltkanten (30) voneinander getrennt sind und derart umklappbar sind, dass eine weitere Tasche (32) zum Aufnehmen der ersten Sterilbarriere (4) zwischen den Lufttaschen (10) gelagert ist.

10. Medizinische Sterilverpackung (1) nach Anspruch 9, **dadurch gekennzeichnet, dass** die beiden Lufttaschen (10) jeweils auf gegenüberliegenden Seiten der weiteren Tasche (32) mit der darin befindlichen ersten Sterilbarriere (4) angeordnet sind.

11. Medizinische Sterilverpackung (1) nach Anspruch 9, **dadurch gekennzeichnet, dass** die beiden Lufttaschen (10) auf der gleichen Seite von der weiteren Tasche (32) mit der darin befindlichen ersten Sterilbarriere (4) angeordnet sind.

12. Medizinische Sterilverpackung (1) nach einem der Ansprüche 9 bis 11, **dadurch gekennzeichnet, dass** die Lufttaschen (10) derart an den Umklapp- oder Faltkanten (30) umgeklappt werden, damit die weitere Tasche (32) von zumindest zwei Seiten von den Lufttaschen (10) umgeben sind.

13. Medizinische Sterilverpackung (1) nach einem der Ansprüche 1 bis 12, **dadurch gekennzeichnet, dass** die Umklapp- oder Faltkante (30) die beiden Membranen (20, 22) der Doppelmembran (19) verbinden und insbesondere durch eine punkt- oder linienförmige Schweißnaht fertigbar sind.

14. System aus einer medizinischen Sterilverpackung (1) nach einem der Ansprüche 1 bis 13 und einem medizinischen Produkt (8), das in der medizinischen Sterilverpackung (1) steril verpackt ist,
**dadurch gekennzeichnet, dass**
die wenigstens zwei Lufttaschen (10) der zweiten Sterilbarriere (6) derart um das medizinische Produkt (8) umgeklappt sind, dass die planare Membran (22) der Doppelmembran (19) der zweiten Sterilbarriere (6) in Richtung des medizinischen Produkts (8) zeigt und der Aufnahmeraum zur Aufnahme der ersten Sterilbarriere (4) in der planaren Membran (22) ausgebildet ist.

15. System nach Anspruch 14, **dadurch gekennzeichnet, dass** die Ausdehnung der planaren Membran (22) größer ist als das medizinische Produkt (8) und der Aufnahmeraum das medizinische Produkt (8) vollständig oder annähernd vollständig umschließt.

## Claims

1. A sterile medical packaging (1) for packaging a medical product (8), comprising:
- a first sterile barrier (4) prepared and configured to receive the medical product (8) in a heat-sealed/sealed manner,
- a second sterile barrier (6) prepared and configured to form a receiving space for receiving the first sterile barrier (4) in a heat-sealed/sealed manner, and
- an outer protective packaging (2) prepared and configured to receive the first sterile barrier (4) and the second sterile barrier (6),
wherein
the second sterile barrier (6) comprises or is a double membrane (19) that forms at least two air pockets (10), which are separated from each other by a predefined fold-down or folding edge (30) in the double membrane (19), whereby, in the folded-down state of the double membrane (19) along the fold-down or folding edge (30), the two air pockets (10) come to lie in alignment with each other in such a way that the receiving space can be created between them.

2. The sterile medical packaging (1) according to claim 1, **characterized in that** the double membrane has an outer membrane (20) that bulges out concavely, and an inner membrane (22) of the double membrane (19) remains planar.

3. The sterile medical packaging (1) according to claim 1 or 2, **characterized in that** when the air pockets (10) are folded down, the planar membrane (22) comes to lie in sections against each other essentially flat.

4. The sterile medical packaging (1) according to claim 2 or 3, **characterized in that** the heat-sealable/sealable receiving space can be created between the planar membrane sections (22), which lie flat against each other, in the area of the aligned air pockets (10), preferably by plastic or elastic deformation of the planar membrane sections (22) which lie flat against each other.

5. The sterile medical packaging (1) according to one of the claims 1 to 4, **characterized in that** the second sterile barrier (6) has exactly two air pockets (10).

6. The sterile medical package (1) according to one of claims 1 to 5, **characterized in that** the first convex membrane (20) of the second sterile barrier (6) is made of a rigid material and the second planar membrane (22) is elastic.

7. The sterile packaging (1) according to one of claims 1 to 6, **characterized in that** the first sterile barrier (4) comprises two films (14, 16) and is prepared and configured to heat-seal/seal the medical product (8) between the two films (14, 16) such that relative movement between the films (14, 16) and the medical product (8) is reduced.

8. The sterile medical packaging (1) according to one of claims 1 to 7, **characterized by** several sealings, wherein a first and a second sealing (18) seal the first sterile barrier (4), a third, a fourth and a fifth sealing (28) each seal the double membrane (19) next to the air pockets (10) of the second sterile barrier (6), and a sixth sealing (24) seals the two air pockets (10) which lie on top of each other together.

9. The sterile medical packaging (1) according to one of claims 1 to 8, **characterized in that** the second sterile barrier (6) has two air pockets (10), which are separated from each other by predefined folding edges (30) and can be folded down in such a way that an additional pocket (32) for receiving the first sterile barrier (4) is positioned between the air pockets (10).

10. The sterile medical packaging (1) according to claim 9, **characterized in that** the two air pockets (10) are each arranged on opposite sides of the additional pocket (32) with the first sterile barrier (4) located therein.

11. The sterile medical packaging (1) according to claim 9, **characterized in that** the two air pockets (10) are arranged on the same side of the additional pocket (32) with the first sterile barrier (4) located therein.

12. The sterile medical packaging (1) according to one of the claims 9 to 11, **characterized in that** the air pockets (10) are folded down on the folding edges (30) in such a way that the additional pocket (32) is surrounded by the air pockets (10) on at least two sides.

13. The sterile medical packaging (1) according to one of the claims 1 to 12, **characterized in that** the folding edge (30) connects the two membranes (20, 22) of the double membrane (19) and can be manufactured in particular by a point-shaped or line-shaped weld seam.

14. A system comprising a sterile medical packaging (1) according to one of claims 1 to 13 and a medical product (8) that is packaged in a sterile manner in the sterile medical packaging (1),
**characterized in that**
the at least two air pockets (10) of the second sterile barrier (6) are folded down around the medical product (8) such that the planar membrane (22) of the double membrane (19) of the second sterile barrier (6) faces toward of the medical product (8) and the receiving space for receiving of the first sterile barrier (4) is configured in the planar membrane (22).

15. The system according to claim 14, **characterized in that** the extent of the planar membrane (22) is greater than that of the medicinal product (8), and the receiving space completely or almost completely encloses the medicinal product (8).

## Revendications

1. Emballage stérile (1) médical pour l'emballage d'un produit médical (8) présentant :
- une première barrière stérile (4) qui est préparée et formée afin de recevoir le produit médical (8) de manière soudée/scellée,
- une seconde barrière stérile (6) qui est préparée et formée afin de former un espace de réception pour la réception de la première barrière stérile (4) de manière soudée/scellée et
- un emballage de protection (2) extérieur qui est préparé et formé afin de recevoir la première barrière stérile (4) et la seconde barrière stérile (6),
dans lequel la seconde barrière stérile (6) présente ou est une membrane double (19) formant au moins deux poches d'air (10), lesquelles sont séparées l'une de l'autre par un bord de rabattement ou de pliage (30) prédéfinie dans la membrane double (19), selon lequel dans l'état rabattu de la membrane double (19) le long du bord de rabattement ou de pliage (30), les deux poches d'air (10) viennent se poser en alignement l'une sur l'autre de telle manière que l'espace de réception puisse être généré au milieu.

2. Emballage stérile (1) médical selon la revendication 1, **caractérisé en ce que** la membrane double présente une membrane extérieure (20) qui se courbe de manière concave et une membrane intérieure (22) de la membrane double (19) reste plane.

3. Emballage stérile (1) médical selon la revendication 1 ou 2, **caractérisé en ce que** lors du rabattement des poches d'air (10), la membrane plane (22) vient se poser par sections sensiblement à plat l'une contre l'autre.

4. Emballage stérile (1) médical selon la revendication 2 ou 3, **caractérisé en ce que** l'espace de réception soudable/scellable peut être généré entre les sections de membrane (22) planes, reposant à plat l'une sur l'autre dans la zone des poches d'air (10) s'alignant, de préférence par déformation plastique ou élastique des sections de membrane (22) planes, reposant à plat l'une sur l'autre.

5. Emballage stérile (1) médical selon l'une quelconque des revendications 1 à 4, **caractérisé en ce que** la seconde barrière stérile (6) présente précisément deux poches d'air (10).

6. Emballage stérile (1) médical selon l'une quelconque des revendications 1 à 5, **caractérisé en ce que** la première membrane convexe (20) de la seconde barrière stérile (6) est fabriquée en un matériau solide et la seconde membrane (22) plane est élastique.

7. Emballage stérile (1) médical selon l'une quelconque des revendications 1 à 6, **caractérisé en ce que** la première barrière stérile (4) présente deux films (14, 16) et est préparée et formée afin de souder/sceller le produit médical (8) entre les deux films (14, 16) de telle manière qu'un mouvement relatif soit réduit entre les films (14, 16) et le produit médical (8).

8. Emballage stérile (1) médical selon l'une quelconque des revendications 1 à 7, **caractérisé par** plusieurs scellements, dans lequel un premier et un deuxième scellement (18) scellent la première barrière stérile (4), un troisième, un quatrième et un cinquième scellement (28) scellent respectivement la double membrane (19) à côté des poches d'air (10) de la seconde barrière stérile (6) et un sixième scellement (24) scelle les deux poches d'air (10) reposant l'une contre l'autre l'une avec l'autre.

9. Emballage stérile (1) médical selon l'une quelconque des revendications 1 à 8, **caractérisé en ce que** la seconde barrière stérile (6) présente deux poches d'air (10) qui sont séparées l'une de l'autre par des bords de rabattement ou de pliage (30) prédéfinis et sont rabattables de telle manière qu'une autre poche (32) soit logée pour la réception de la première barrière stérile (4) entre les poches d'air (10).

10. Emballage stérile (1) médical selon la revendication 9, **caractérisé en ce que** les deux poches d'air (10) sont agencées respectivement sur des côtés opposés de l'autre poche (32) avec la première barrière stérile (4) se trouvant dedans.

11. Emballage stérile (1) médical selon la revendication 9, **caractérisé en ce que** les deux poches d'air (10) sont agencées sur le même côté de l'autre poche (32) avec la première barrière stérile (4) se trouvant dedans.

12. Emballage stérile (1) médical selon l'une quelconque des revendications 9 à 11, **caractérisé en ce que** les poches d'air (10) sont rabattues au niveau des bords de rabattement ou de pliage (30) afin que l'autre poche (32) soit entourée par au moins deux côtés par les poches d'air (10).

13. Emballage stérile (1) médical selon l'une quelconque des revendications 1 à 12, **caractérisé en ce que** les bords de rabattement ou de pliage (30) relient les deux membranes (20, 22) de la double membrane (19) et peuvent être terminés en particulier par un cordon de soudure ponctuel ou linéaire.

14. Système composé d'un emballage stérile (1) médical selon l'une quelconque des revendications 1 à 13 et d'un produit médical (8) qui est emballé de manière stérile dans l'emballage stérile (1) médical,
**caractérisé en ce que**
les au moins deux poches d'air (10) de la seconde barrière stérile (6) sont rabattues autour du produit médical (8) de telle manière que la membrane (22) plane de la double membrane (19) de la seconde barrière stérile (6) soit dirigée en direction du produit médical (8) et l'espace de réception est formé pour la réception de la première barrière stérile (4) dans la membrane (22) plane.

15. Système selon la revendication 14, **caractérisé en ce que** l'extension de la membrane plane (22) est supérieure au produit médical (8) et l'espace de réception entoure complètement ou approximativement complètement le produit médical (8).
